# EUROPEAN PATENT APPLICATION

(11) **EP 4 796 319 A1**
(43) Date of publication of application: **26.08.2026**
(21) Application number: 25158882.8
(22) Date of filing: 19.02.2025
(51) Int. Cl.: B32B 27/08, B32B 27/30, B32B 27/40, C08J 5/18, C08J 7/04, C08L 33/08, C08L 75/04, A61K 8/81, A61Q 19/08, C08K 3/34, A61K 8/87, A61K 8/02, A45D 44/00

(54) **MEMORY FILM FOR A SKIN**

(71) Applicant: Amorepacific Corporation, Seoul 04386 (KR); SanaHeal, Inc., Somerville MA 02143 (US)
(72) Inventor: Seo, Jeongeun, Yongin-si (KR); Kim, Jihoon, Yongin-si (KR); Pi, Bongsoo, Yongin-si (KR); Han, Kyungsup, Yongin-si (KR); Yuk, Hyunwoo, Boston (US); Zheng, Yi, Boston (US)
(74) Representative: Schön, Christoph

(57) **Abstract**

The present specification relates to a memory film that not only has excellent mechanical properties but also outstanding elasticity, maintains an elongated state during stretching and drying, and contracts the skin with its pre-memorized contractile force when attached to moist skin, and has the effect of enhancing skin elasticity, improving skin wrinkles, increasing skin density, and reducing pore depth, pore area, pore count, and pore volume.

## Description

### BACKGROUND OF THE INVENTION

### [Field of the Invention]

The present specification discloses a film that exhibits excellent mechanical properties and elasticity, maintains an elongated state during stretching and drying, and contracts the skin due to its pre-memorized contractile force when attached to moist skin.

### [Description of the Related Art]

To deliver the force that contracts the skin, there are inconveniences such as requiring an applicator or manual stretching during attachment, making it difficult to use with consistent force each time. Additionally, conventional films attached to the skin have the drawback of losing attachment ability in moist environments.

### SUMMARY OF THE INVENTION

In one aspect of the present disclosure, there is provided a film that exhibits excellent mechanical properties and elasticity, maintains an elongated state during stretching and drying, and contracts the skin with its pre-memorized contractile force when attached to moist skin.

In one aspect, the present disclosure provides a memory film that includes a first layer including acrylic acid and polyurethane, which is attached to the skin and then contracts to a memorized rate after a period of time.

In another aspect, the present disclosure provides a method of manufacturing the aforementioned memory film, which includes: applying and drying a solution including acrylic acid and polyurethane; and stretching the applied and dried material.

In one aspect, the memory film according to the present disclosure exhibits excellent mechanical properties and elasticity, maintains an elongated state during stretching and drying, and can contract the skin with its pre-memorized contractile force when attached to moist skin.

### BRIEF DESCRIPTION OF THE DRAWINGS

FIG. 1 illustrates the contraction of a memory film in a moist environment, according to an embodiment of the present disclosure.
FIG. 2 is a photograph observing the movement of the skin caused by contraction when a memory film according to an embodiment of the present disclosure is attached to the skin.
FIG. 3 illustrates the results of comparing the physical properties of a dual memory film and a single memory film according to an embodiment of the present disclosure.

### DETAILED DESCRIPTION OF THE INVENTION

Hereinafter, the present disclosure will be described in more detail with reference to the following embodiments. However, the following embodiments are provided for illustrative purposes only to assist in understanding the present disclosure and are not intended to limit the scope and range of the present disclosure.

In exemplary embodiments of the present disclosure, a memory film is provided, which includes a first layer including acrylic acid and polyurethane, which is attached to the skin and then contracts to a memorized rate after a period of time.

In exemplary embodiments of the present disclosure, a method is provided for attaching a memory film, which includes a first layer including acrylic acid and polyurethane, to the skin and contracting the memory film at a memorized ratio after a period of time.

In exemplary embodiments of the present disclosure, there is provided a use of a first layer including acrylic acid and polyurethane for manufacturing a memory film that is attached to the skin and then contracts to a memorized rate after a period of time.

In one embodiment, the memory film may be for enhancing skin elasticity.

In one embodiment, the memory film may be for improving skin wrinkles.

In one embodiment, the memory film may be for increasing skin density.

In one embodiment, the memory film may be for reducing one or more of pore depth, pore area, pore count, and pore volume.

In one embodiment, the time may be 1 to 24 hours.

For example, the time may be 1 hour or more, 5 hours or more, 10 hours or more, 15 hours or more, or 20 hours or more, and may be 24 hours or less, 20 hours or less, 15 hours or less, 10 hours or less, or 5 hours or less.

In one embodiment, the memorized ratio may be such that a surface area after a period of time is 100 % relative to 150 to 200 % of the surface area at the time of skin attachment.

For example, the surface area after a period of time may be 100 % relative to 150 % or more, 170 % or more, or 190 % or more, and 200 % or less, 180 % or less, or 160 % or less of the surface area at the time of skin attachment.

In one embodiment, the acrylic acid may be polyacrylic acid.

In one embodiment, the content of polyurethane may be less than 50 wt% based on a total weight of the first layer. When the content of polyurethane is 50 wt% or more, the strength of the physical properties may increase, but it may return to its original state during stretching and drying, making it difficult to use as a memory film.

For example, the content of polyurethane may be less than 50 wt%, less than 30 wt%, less than 20 wt%, less than 10 wt%, less than 5 wt%, or less than 1 wt%, based on the total weight of the first layer. For example, it may be 1 wt% or more, 5 wt% or more, 10 wt% or more, 20 wt% or more, 30 wt% or more, or 40 wt% or more.

In one embodiment, the weight ratio of acrylic acid to polyurethane may be 1 to 3:1.

For example, the weight ratio of acrylic acid to polyurethane may be 1:1 or more, 1.5:1 or more, 2:1 or more, or 2.5:1 or more, and may be 3:1 or less, 2.5:1 or less, 2:1 or less, or 1.5:1 or less.

In one embodiment, the first layer may further include Laponite. Including Laponite may further enhance the strength of the memory film.

The total molecular weight of the acrylic acid and polyurethane may be 100,000 to 1,000,000.

In the present specification, a molecular weight is defined as a weight average molecular weight (Mw).

For example, the total molecular weight of acrylic acid and polyurethane may be 100,000 or more, 200,000 or more, 300,000 or more, 400,000 or more, 500,000 or more, 600,000 or more, 700,000 or more, 800,000 or more, or 900,000 or more, and may be 1,000,000 or less, 900,000 or less, 800,000 or less, 700,000 or less, 600,000 or less, 500,000 or less, 400,000 or less, 300,000 or less, or 200,000 or less.

In one embodiment, a thickness of the first layer may be 100 to 3000 µm.

For example, the thickness of the first layer may be 100 µm or more, 300 µm or more, 500 µm or more, 700 µm or more, 900 µm or more, 1100 µm or more, 1300 µm or more, 1500 µm or more, 1700 µm or more, 2000 µm or more, or 2500 µm or more, and may be 3000 µm or less, 2500 µm or less, 2000 µm or less, 1700 µm or less, 1500 µm or less, 1300 µm or less, 1100 µm or less, 900 µm or less, 700 µm or less, 500 µm or less, or 300 µm or less.

In one embodiment, the memory film may further include a second layer including one or more selected from the group consisting of polyurethane, spandex, PVC, and nylon. The memory film may further include a second layer, the second layer being a stretchable mesh or film, such as polyurethane, spandex, PVC, or nylon, to increase the physical properties and contraction strength of the memory film.

In one embodiment, the second layer may include polyurethane.

For example, the second layer may be manufactured by applying a solution including polyurethane, and the content of polyurethane in the solution may be less than 50 wt%, less than 30 wt%, less than 20 wt%, less than 10 wt%, less than 5 wt%, or less than 1 wt%, based on the total weight of the solution. For instance, it may be 1 wt% or more, 5 wt% or more, 10 wt% or more, 20 wt% or more, 30 wt% or more, or 40 wt% or more.

For example, in case of the finally manufactured second layer, the solvent in the solution may be completely volatilized, resulting in the layer being composed of polyurethane.

In one embodiment, a thickness of the second layer may be 1 to 200 µm.

For example, the thickness of the second layer may be 1 µm or more, 30 µm or more, 50 µm or more, 70 µm or more, 90 µm or more, 100 µm or more, 130 µm or more, 150 µm or more, or 170 µm or more, and may be 200 µm or less, 170 µm or less, 150 µm or less, 130 µm or less, 100 µm or less, 90 µm or less, 70 µm or less, 50 µm or less, 30 µm or less, 10 µm or less, or 5 µm or less.

In another exemplary embodiment of the present disclosure, a method of manufacturing the aforementioned memory film is provided. The method includes: applying and drying a solution including acrylic acid and polyurethane; and stretching the applied and dried material.

In one embodiment, the solution including acrylic acid and polyurethane may further include Laponite.

In one embodiment, the solution including acrylic acid and polyurethane may further include water and ethanol.

In one embodiment, prior to the applying and drying of the solution including acrylic acid and polyurethane, the method may further include applying and drying a solution including one or more selected from the group consisting of polyurethane, spandex, PVC, and nylon, thereby manufacturing a memory film further including a second layer.

In one embodiment, prior to the applying and drying of the solution including acrylic acid and polyurethane, the method may further include applying and drying a solution including polyurethane, thereby manufacturing a memory film further including a second layer.

For example, based on the total weight of the solution including polyurethane, the content of polyurethane may be less than 50 wt%, less than 30 wt%, less than 20 wt%, less than 10 wt%, less than 5 wt%, or less than 1 wt%, and may be 1 wt% or more, 5 wt% or more, 10 wt% or more, 20 wt% or more, 30 wt% or more, or 40 wt% or more.

In one embodiment, the method may further include a drying step after the stretching step.

Hereinafter, the present invention will be described in more detail through embodiments. These embodiments are just illustrative of the present invention, and it is apparent to one of ordinary skill in the art that the scope of the present invention is not to be interpreted as limited by these embodiments.

### Example

### Manufacturing Example 1 - Manufacturing of 1-Layer Memory Film

1) Prepare Solution A.
   - Dissolve 10 to 15 wt% Polyurethane in a 95 % ethanol solution.
2) Prepare Solution B.
   - Dissolve Laponite into a 1 to 5 wt% polyacrylic acid solution. The total molecular weight of polyacrylic acid and polyurethane is 800,000.
3) Mix Solution A, Solution B, and 70 % ethanol using a mixer. That is, a PU-PAA-Laponite resin is prepared.
4) Evenly apply the well-mixed PU-PAA-Laponite resin in 3) above to a thickness of 500 to 2000 µm and dry it.
5) Perform secondary heating and drying at a temperature of 100 °C or higher to induce crosslinking reactions caused by Laponite.
6) Hydrate the dried film, stretch it to 150 to 200 %, and then heat it to completely dry.

### Manufacturing Example 2 - Manufacturing of a 2-Layer Memory Film (Double Film, Double Patch)

1) Prepare Solution A.
   - Dissolve 10 to 15 wt% polyurethane in a 95 % ethanol solution.
2) Apply Solution A to a thickness of 50 to 100 µm and dry it.
3) Prepare Solution B.
   - Dissolve Laponite into a 1 to 5 wt% polyacrylic acid solution.
4) Mix Solution A, Solution B, and 70 % ethanol using a mixer. That is, a PU-PAA-Laponite resin is prepared.
5) Evenly apply the well-mixed PU-PAA-Laponite resin in 3) above onto the film where Solution A has been dried, at a thickness of 500 to 2000 µm, and dry it.
6) Perform secondary heating and drying at a temperature of 100 °C or higher to induce crosslinking reactions caused by Laponite.
7) Hydrate the dried film, stretch it to 150 to 200 %, and then heat it to completely dry.

### Test Example

### Test Example 1 - Confirmation of Contraction in a Moist Environment (Providing Strain)

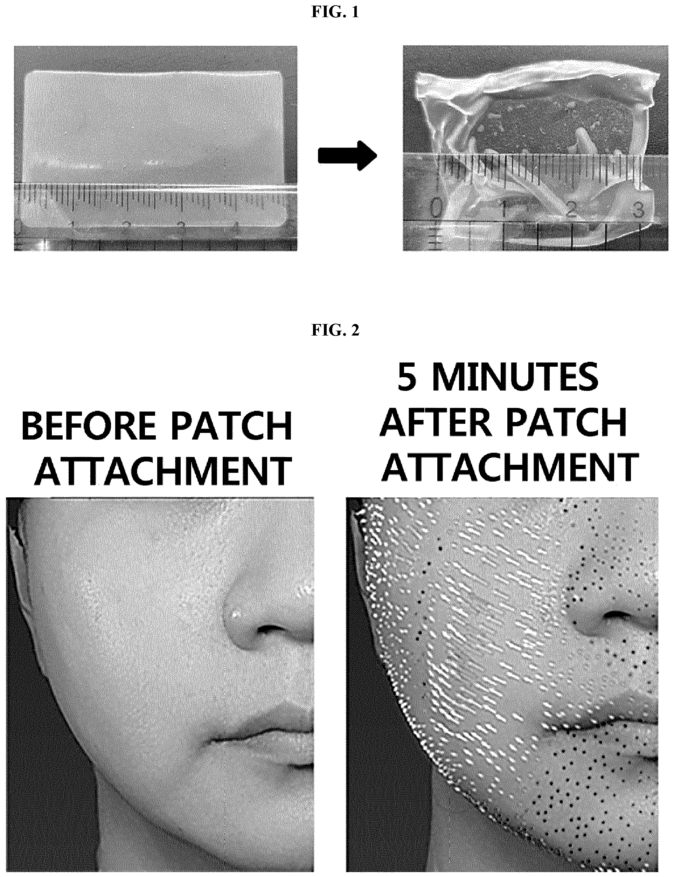

As illustrated in FIG. 1, when a stretched and dried film (5 cm) was provided with a moist environment (water), it was confirmed that the size of the film decreased (3.1 cm).

### Test Example 2 - Facial Contour Changes Upon Skin Attachment

After applying an appropriate amount of water to the cheek area of the face and attaching a 5x3 cm film, it was confirmed using VECTRA H2 FACE Sculptor^{®} that the film contracted and altered the facial contour 5 minutes after application.

With reference to FIG. 2, compared to the left side of the face without the film, it was confirmed that the skin around the area where the film was attached on the right side of the face gathered.

### Test Example 3 - Comparison of Physical Properties Based on Dual or Single Film and Molecular Weight

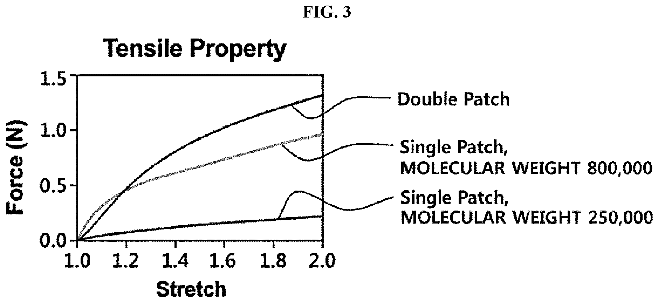

With reference to FIG. 3, in case of the dual film (double patch) manufactured with a total molecular weight of polyacrylic acid and polyurethane of Mw = 800,000, the tensile property was the highest. In case of the single film (single patch), the tensile strength was significantly higher when the total molecular weight (Mw) of polyacrylic acid and polyurethane was 800,000 compared to 250,000.

### Test Example 4 - Human Efficacy Evaluation for Multiple Items Beyond Elasticity

● **Test Product:** Using the memory film manufactured in Manufacturing Example 1, evaluations were conducted on elasticity, lifting, wrinkles, skin density, and pores.
● **Test Subjects:** A total of 25 women who met the inclusion criteria and did not fall under the exclusion criteria were selected [final test completed by 20 participants, aged 47.200 ± 3.548, with 5 dropouts (non-compliance with the schedule)].
● **Test Method:** The test product was attached to the forehead & glabella, and nasolabial folds on the face before sleeping. After approximately 8 hours of sleep, it was gently removed while washing the face in the morning. After continuous use for 7 days, device measurements and survey evaluations were conducted before using the test product, 4 days after use, 7 days after use, and 21 days after discontinuation.
   * Use the product once in the screening phase before starting the test and identify adverse reactions. The test is conducted on individuals who showed no adverse reactions.
● **Evaluation Items**

### 1) Device evaluation

- Elasticity: Cutometer
- Lifting: F-ray
- Wrinkles: Primos
- Skin Density: Skin scanner
- Pores: Antera

### 2) Subjective Survey Evaluation Based on Feelings of Use: Subjective survey evaluation conducted by test subjects.

### 3) Safety Evaluation: Evaluation of adverse reactions reported by test subjects.

### ● Test results

This test targeted women aged 40 to 53 with wrinkles and pores (who had not undergone any procedures within the past 3 months) to measure improvements in multiple items, including elasticity. The test product was used for 7 days, and both device measurements and subjective survey evaluations based on feelings of use were conducted, confirming the following results.

### 1) Elasticity, R2

### 1-1) Forehead

- As shown in Table 1 below, it was confirmed that elasticity measurement values significantly increased compared to before using the product, showing an improvement of 10.000 % after 4 days of use, 12.857 % after 7 days of use, and 8.750 % after 21 days of discontinuation, all at a significant level (p<0.05).

**[Table 1]**

| | | **Before use** | **After 4 days of use** | **After 7 days of use** | **21 days after discontinuation** |
|---|---|---|---|---|---|
| **Elasticity** | **Average** | 0.560 | 0.616 | 0.632 | 0.609 |
| **measurement value (R2)** | **Standard Deviation** | 0.057 | 0.055 | 0.057 | 0.059 |
| **Change rate^{a} (%)** | **Before use - After 4 days of use** | 10.000 | | | |
| | **Before use - After 7 days of use** | 12.857 | | | |
| **Change rate ^{b} (%)** | **Before use - 21 days after discontinuation** | 8.750 | | | |
| **Change rate^{c} (%)** | **After 7 days of use - 21 days after discontinuation** | -3.639 | | | |
| **Significance probability (p-value)** | **Before use - After 4 days of use** | <0.001** | | | |
| | **Before use - After 7 days of use** | <0.001** | | | |
| | **Before use - 21 days after discontinuation** | <0.001** | | | |
| | **After 7 days of use - 21 days after discontinuation** | 0.034* | | | |
| **Number of improved test subjects/%** | **Before use - After 4 days of use** | 20 individuals / 100.000 % | | | |
| | **Before use - After 7 days of use** | 20 individuals / 100.000 % | | | |
| | **Before use - 21 days after discontinuation** | 20 individuals / 100.000 % | | | |

| | | | | | |
|---|---|---|---|---|---|
| *** : *P*<0.05 by Paired samples t-test** ****: *P*<0.05 by repeated measures ANOVA** Change ratea (%): [(measurement value after use - measurement value before use)/ measurement value before use × 100] Change rateb (%): [(measurement value 21 days after discontinuation - measurement value before use)/measurement value before use × 100] Change ratec (%): [(measurement value 21 days after discontinuation - measurement value after 7 days of use)/measurement value after 7 days of use × 100] | | | | | |

### 1-2) Cheek

- As shown in Table 2 below, it was confirmed that elasticity measurement values significantly increased compared to before using the product, showing an improvement of 7.431 % after 4 days of use, 9.370 % after 7 days of use, and 7.916 % after 21 days of discontinuation, all at a significant level (p<0.05).
- The elasticity measurement value 21 days after discontinuation did not show a significant difference compared to 7 days after use (p>0.05), confirming the effect of elasticity maintenance up to 21 days after discontinuation.

**[Table 2]**

| | | **Before use** | **After 4 days of use** | **After 7 days of use** | **21 days after discontinuation** |
|---|---|---|---|---|---|
| **Elasticity measurement value (R2)** | **Average** | 0.619 | 0.665 | 0.677 | 0.668 |
| | **Standard Deviation** | 0.076 | 0.081 | 0.076 | 0.071 |
| **Change rate^{a} (%)** | **Before use - After 4 days of use** | 7.431 | | | |
| | **Before use - After 7 days of use** | 9.370 | | | |
| **Change rate ^{b} (%)** | **Before use - 21 days after discontinuation** | 7.916 | | | |
| **Change rate^{c} (%)** | **After 7 days of use - 21 days after discontinuation** | -1.329 | | | |
| **Significance probability (*p*-value)** | **Before use - After 4 days of use** | <0.001^{##} | | | |
| | **Before use - After 7 days of use** | <0.001^{##} | | | |
| | **Before use - 21 days after discontinuation** | <0.001^{##} | | | |
| | **After 7 days of use - 21 days after discontinuation** | 0.162 | | | |
| **Number of improved test subjects/%** | **Before use - After 4 days of use** | 18 individuals / 90.000 % | | | |
| | **Before use - After 7 days of use** | 20 individuals / 100.000 % | | | |
| | **Before use - 21 days after discontinuation** | 20 individuals / 100.000 % | | | |

| | | | | | |
|---|---|---|---|---|---|
| **^{##}: *P*<0.025(=0.05/2) by Firedman test, post hoc Wilcoxon signed rank test with Bonferroni correction** Change ratea (%): [(measurement value after use - measurement value before use)/ measurement value before use × 100] Change rateb (%): [(measurement value 21 days after discontinuation - measurement value before use)/measurement value before use × 100] Change ratec (%): [(measurement value 21 days after discontinuation - measurement value after 7 days of use)/measurement value after 7 days of use × 100] | | | | | |

### 2) Lifting, °

- As shown in Table 3 below, it was confirmed that lifting measurement values significantly increased compared to before using the product, showing an improvement of 1.508 % after 4 days of use, 2.364 % after 7 days of use, and 0.724 % after 21 days of discontinuation, all at a significant level (p<0.05).

**[Table 3]**

| | | **Before use** | **After 4 days of use** | **After 7 days of use** | **21 days after discontinuation** |
|---|---|---|---|---|---|
| **Lifting Measurement value (°)** | **Average** | 55.250 | 56.083 | 56.556 | 55.650 |
| | **Standard Deviation** | 9.353 | 9.230 | 9.273 | 9.258 |
| **Change rate^{a} (%)** | **Before use - After 4 days of use** | 1.508 | | | |
| | **Before use - After 7 days of use** | 2.364 | | | |
| **Change rate ^{b} (%)** | **Before use - 21 days after discontinuation** | 0.724 | | | |
| **Change rate^{c} (%)** | **After 7 days of use - 21 days after discontinuation** | -1.602 | | | |
| **Significance probability (*p*-value)** | **Before use - After 4 days of use** | <0.001** | | | |
| | **Before use - After 7 days of use** | <0.001** | | | |
| | **Before use - 21 days after discontinuation** | 0.014** | | | |
| | **After 7 days of use - 21 days after discontinuation** | <0.001* | | | |
| **Number of improved test subjects/%** | **Before use - After 4 days of use** | 20 individuals / 100.000 % | | | |
| | **Before use - After 7 days of use** | 19 individuals / 95.000 % | | | |
| | **Before use - 21 days after discontinuation** | 17 individuals / 85.000 % | | | |

| | | | | | |
|---|---|---|---|---|---|
| *** : *P*<0.05 by Paired samples t-test** ****: *P*<0.05 by repeated measures ANOVA** Change ratea (%): [(measurement value after use - measurement value before use)/ measurement value before use × 100] Change rateb (%): [(measurement value 21 days after discontinuation - measurement value before use)/measurement value before use × 100] Change ratec (%): [(measurement value 21 days after discontinuation - measurement value after 7 days of use)/measurement value after 7 days of use × 100] | | | | | |

### 3) Wrinkles, Ra

### 3-1) Forehead

- As shown in Table 4 below, the wrinkle measurement value decreased by 7.968 % at a significant level (*p*<0.05) after 4 days of use, confirming an improvement compared to before using the product.

**[Table 4]**

| | | **Before use** | **After 4 days of use** | **After 7 days of use** | **21 days after discontinuation** |
|---|---|---|---|---|---|
| **Wrinkle measurement value (Ra)** | **Average** | 23.142 | 21.298 | 22.161 | 22.693 |
| | **Standard Deviation** | 5.083 | 4.745 | 3.813 | 4.568 |
| **Change rate^{a} (%)** | **Before use - After 4 days of use** | -7.968 | | | |
| | **Before use - After 7 days of use** | -4.239 | | | |
| **Change rate ^{b} (%)** | **Before use - 21 days after discontinuation** | -1.940 | | | |
| **Change rate^{c} (%)** | **After 7 days of use - 21 days after discontinuation** | 2.401 | | | |
| **Significance probability (*p*-value)** | **Before use - After 4 days of use** | <0.001^{##} | | | |
| | **Before use - After 7 days of use** | 0.191 | | | |
| | **Before use - 21 days after discontinuation** | 0.135 | | | |
| | **After 7 days of use - 21 days after discontinuation** | 0.351 | | | |
| **Number of improved test subjects/%** | **Before use - After 4 days of use** | 17 individuals / 85.000 % | | | |
| | **Before use - After 7 days of use** | 13 individuals / 65.000 % | | | |
| | **Before use - 21 days after discontinuation** | 15 individuals / 75.000 % | | | |

| | | | | | |
|---|---|---|---|---|---|
| **^{##}: *P*<0.025(=0.05/2) by Firedman test, post hoc Wilcoxon signed rank test with Bonferroni correction** Change ratea (%): [(measurement value after use - measurement value before use)/ measurement value before use × 100] Change rateb (%): [(measurement value 21 days after discontinuation - measurement value before use)/measurement value before use × 100] Change ratec (%): [(measurement value 21 days after discontinuation - measurement value after 7 days of use)/measurement value after 7 days of use × 100] | | | | | |

### 3-2) Glabella

- As shown in Table 5 below, the wrinkle measurement value decreased by 5.682 % at a significant level (p<0.05) after 4 days of use, confirming an improvement compared to before using the product.

**[Table 5]**

| | | **Before use** | **After 4 days of use** | **After 7 days of use** | **21 days after discontinuation** |
|---|---|---|---|---|---|
| **Wrinkle measurement value (Ra)** | **Average** | 17.636 | 16.634 | 17.501 | 17.404 |
| | **Standard Deviation** | 2.871 | 2.768 | 2.857 | 2.870 |
| **Change rate^{a} (%)** | **Before use - After 4 days of use** | -5.682 | | | |
| | **Before use - After 7 days of use** | -0.765 | | | |
| **Change rate ^{b} (%)** | **Before use - 21 days after discontinuation** | -1.315 | | | |
| **Change rate^{c} (%)** | **After 7 days of use - 21 days after discontinuation** | -0.554 | | | |
| **Significance probability (*p*-value)** | **Before use - After 4 days of use** | 0.002^{##} | | | |
| | **Before use - After 7 days of use** | 0.313 | | | |
| | **Before use - 21 days after discontinuation** | 0.411 | | | |
| | **After 7 days of use - 21 days after discontinuation** | 0.819 | | | |
| **Number of improved test subjects/%** | **Before use - After 4 days of use** | 18 individuals / 90.000 % | | | |
| | **Before use - After 7 days of use** | 14 individuals / 70.000 % | | | |
| | **Before use - 21 days after discontinuation** | 11 individuals / 55.000 % | | | |

| | | | | | |
|---|---|---|---|---|---|
| ***: *P*<0.05 by Paired samples t-test** ****: *P*<0.05 by repeated measures ANOVA** Change ratea (%): [(measurement value after use - measurement value before use)/ measurement value before use × 100] Change rateb (%): [(measurement value 21 days after discontinuation - measurement value before use)/measurement value before use × 100] Change ratec (%): [(measurement value 21 days after discontinuation - measurement value after 7 days of use)/measurement value after 7 days of use × 100] | | | | | |

### 3-3) Nasolabial Folds

- As shown in Table 6 below, the wrinkle measurement value decreased by 2.907 % after 4 days of use, and 3.160 % after 7 days of use at a significant level (p<0.05), confirming an improvement compared to before using the product.

**[Table 6]**

| | | **Before use** | **After 4 days of use** | **After 7 days of use** | **21 days after discontinuation** |
|---|---|---|---|---|---|
| **Wrinkle measurement value (Ra)** | **Average** | 21.360 | 20.739 | 20.685 | 21.246 |
| | **Standard Deviation** | 3.181 | 3.412 | 3.002 | 3.427 |
| **Change rate^{a} (%)** | **Before use - After 4 days of use** | -2.907 | | | |
| | **Before use - After 7 days of use** | -3.160 | | | |
| **Change rate ^{b} (%)** | **Before use - 21 days after discontinuation** | -0.534 | | | |
| **Change rate^{c} (%)** | **After 7 days of use - 21 days after discontinuation** | 2.712 | | | |
| **Significance probability (*p*-value)** | **Before use - After 4 days of use** | 0.010** | | | |
| | **Before use - After 7 days of use** | 0.014** | | | |
| | **Before use - 21 days afterdiscontinuation** | 0.718 | | | |
| | **After 7 days of use - 21 days after discontinuation** | 0.082 | | | |
| **Number of improved test subjects/%** | **Before use - After 4 days of use** | 15 individuals / 75.000 % | | | |
| | **Before use - After 7 days of use** | 14 individuals / 70.000 % | | | |
| | **Before use - 21 days after discontinuation** | 14 individuals / 70.000 % | | | |

| | | | | | |
|---|---|---|---|---|---|
| ***: *P*<0.05 by Paired samples t-test** ****: *P*<0.05 by repeated measures ANOVA** Change ratea (%): [(measurement value after use - measurement value before use)/ measurement value before use × 100] Change rateb (%): [(measurement value 21 days after discontinuation - measurement value before use)/measurement value before use × 100] Change ratec (%): [(measurement value 21 days after discontinuation - measurement value after 7 days of use)/measurement value after 7 days of use × 100] | | | | | |

### 4) Skin Density, %.

### 4-1) Forehead

- As shown in Table 7 below, the skin density measurement value increased by 5.028 % after 4 days of use, and 4.470 % after 7 days of use at a significant level (p<0.05), confirming an improvement compared to before using the product.

**[Table 7]**

| | | **Before use** | **After 4 days of use** | **After 7 days of use** | **21 days after discontinuation** |
|---|---|---|---|---|---|
| **Skin density measurement value (Ra)** | **Average** | 69.752 | 73.259 | 72.870 | 69.721 |
| | **Standard Deviation** | 5.049 | 5.151 | 4.964 | 5.317 |
| **Change rate^{a} (%)** | **Before use - After 4 days of use** | 5.028 | | | |
| | **Before use - After 7 days of use** | 4.470 | | | |
| **Change rate ^{b} (%)** | **Before use - 21 days after discontinuation** | -0.044 | | | |
| **Change rate^{c} (%)** | **After 7 days of use - 21 days after discontinuation** | -4.321 | | | |
| **Significance probability (*p*-value)** | **Before use - After 4 days of use** | <0.001** | | | |
| | **Before use - After 7 days of use** | <0.001** | | | |
| | **Before use - 21 days after discontinuation** | 0.950 | | | |
| | **After 7 days of use - 21 days after discontinuation** | <0.001* | | | |
| **Number of improved test subjects/%** | **Before use - After 4 days of use** | 18 individuals / 90.000 % | | | |
| | **Before use - After 7 days of use** | 19 individuals / 95.000 % | | | |
| | **Before use - 21 days after discontinuation** | 12 individuals / 60.000 % | | | |

| | | | | | |
|---|---|---|---|---|---|
| *** : *P*<0.05 by Paired samples t-test** ****: P<0.05 by repeated measures ANOVA** Change ratea (%): [(measurement value after use - measurement value before use)/ measurement value before use × 100] Change rateb (%): [(measurement value 21 days after discontinuation - measurement value before use)/measurement value before use × 100] Change ratec (%): [(measurement value 21 days after discontinuation - measurement value after 7 days of use)/measurement value after 7 days of use × 100] | | | | | |

### 4-2) Cheek

- As shown in Table 8 below, the skin density measurement value increased by both of 7.508 % after 4 days of use, and 7.606 % after 7 days of use at a significant level (p<0.05), confirming an improvement compared to before using the product.

**[Table 8]**

| | | **Before use** | **After 4 days ofuse** | **After 7 days of use** | **21 days after discontinuation** |
|---|---|---|---|---|---|
| **Skin density measurement value (Ra)** | **Average** | 68.383 | 73.517 | 73.584 | 69.734 |
| | **Standard Deviation** | 6.221 | 6.844 | 5.129 | 6.117 |
| **Change rate^{a} (%)** | **Before use - After 4 days of use** | 7.508 | | | |
| | **Before use - After 7 days of use** | 7.606 | | | |
| **Change rate ^{b} (%)** | **Before use - 21 days after discontinuation** | 1.976 | | | |
| **Change rate^{c} (%)** | **After 7 days of use - 21 days after discontinuation** | -5.232 | | | |
| **Significance probability (*p*-value)** | **Before use - After 4 days of use** | <0.001** | | | |
| | **Before use - After 7 days of use** | <0.001** | | | |
| | **Before use - 21 days after discontinuation** | 0.057 | | | |
| | **After 7 days of use - 21 days after discontinuation** | <0.001* | | | |
| **Number of improved test subjects/%** | **Before use - After 4 days of use** | 20 individuals / 100.000 % | | | |
| | **Before use - After 7 days of use** | 18 individuals / 90.000 % | | | |
| | **Before use - 21 days after discontinuation** | 16 individuals / 80.000 % | | | |

| | | | | | |
|---|---|---|---|---|---|
| *** : *P*<0.05 by Paired samples t-test** ****: *P*<0.05 by repeated measures ANOVA** Change ratea (%): [(measurement value after use - measurement value before use)/ measurement value before use × 100] Change rateb (%): [(measurement value 21 days after discontinuation - measurement value before use)/measurement value before use × 100] Change ratec (%): [(measurement value 21 days after discontinuation - measurement value after 7 days of use)/measurement value after 7 days of use × 100] | | | | | |

### 5) Pores

### 5-1) Pore depth, mm

- As shown in Table 9 below, the pore depth value decreased by 10.000 % at a significant level (p<0.05) after 7 days of use, confirming an improvement compared to before using the product.

**[Table 9]**

| | | **Before use** | **After 4 days of use** | **After 7 days of use** | **21 days after discontinuation** |
|---|---|---|---|---|---|
| **Pore depth measurement value (mm)** | **Average** | 0.030 | 0.028 | 0.027 | 0.030 |
| | **Standard Deviation** | 0.010 | 0.007 | 0.007 | 0.008 |
| **Change rate^{a} (%)** | **Before use - After 4 days of use** | -6.667 | | | |
| | **Before use - After 7 days of use** | -10.000 | | | |
| **Change rate ^{b} (%)** | **Before use - 21 days after discontinuation** | 0.000 | | | |
| **Change rate^{c} (%)** | **After 7 days of use - 21 days after discontinuation** | 11.111 | | | |
| **Significance probability (*p*-value)** | **Before use - After 4 days of use** | 0.111 | | | |
| | **Before use - After 7 days of use** | 0.017^{##} | | | |
| | **Before use - 21 days after discontinuation** | 0.570 | | | |
| | **After 7 days of use - 21 days after discontinuation** | 0.006^{#} | | | |
| **Numb er of improved test subjects/%** | **Before use - After 4 days of use** | 13 individuals / 65.000 % | | | |

| | | | | | |
|---|---|---|---|---|---|
| **^{#}: *P*<0.05 by Wilcoxon signed rank test** **^{##}: *P*<0.025(=0.05/2) by Firedman test, post hoc Wilcoxon signed rank test with Bonferroni correction** Change ratea (%): [(measurement value after use - measurement value before use)/ measurement value before use × 100] Change rateb (%): [(measurement value 21 days after discontinuation - measurement value before use)/measurement value before use × 100] Change ratec (%): [(measurement value 21 days after discontinuation - measurement value after 7 days of use)/measurement value after 7 days of use × 100] | | | | | |

### 5-2) Pore area, mm²

- As shown in Table 10 below, it was confirmed that pore area measurement values significantly decreased compared to before using the product, showing an improvement of 15.198 % after 4 days of use, 20.629 % after 7 days of use, and 10.172 % after 21 days of discontinuation, all at a significant level (p<0.05).

**[Table 10]**

| | | **Before use** | **After 4 days of use** | **After 7 days of use** | **21 days after discontinuation** |
|---|---|---|---|---|---|
| **Pore area measurement value (mm²)** | **Average** | 20.990 | 17.800 | 16.660 | 18.855 |
| | **Standard Deviation** | 14.113 | 12.850 | 12.049 | 12.367 |
| **Change rate^{a} (%)** | **Before use - After 4 days of use** | -15.198 | | | |
| | **Before use - After 7 days of use** | -20.629 | | | |
| **Change rate ^{b} (%)** | **Before use - 21 days after discontinuation** | -10.172 | | | |
| **Change rate^{c} (%)** | **After 7 days of use - 21 days after discontinuation** | 13.175 | | | |
| **Significance probability (*p*-value)** | **Before use - After 4 days of use** | <0.001^{##} | | | |
| | **Before use - After 7 days of use** | <0.001^{##} | | | |
| | **Before use - 21 days after discontinuation** | 0.001^{##} | | | |
| | **After 7 days of use - 21 days after discontinuation** | <0.001^{##} | | | |
| **Number of improved test subjects/%** | **Before use - After 4 days of use** | 19 individuals / 95.000 % | | | |
| | **Before use - After 7 days of use** | 20 individuals / 100.000 % | | | |
| | **Before use - 21 days after discontinuation** | 17 individuals / 85.000 % | | | |

| | | | | | |
|---|---|---|---|---|---|
| **: *P*<0.05 by Wilcoxon signed rank test** **^{##}: *P*<0.025(=0.05/2) by Firedman test, post hoc Wilcoxon signed rank test with Bonferroni correction** Change ratea (%): [(measurement value after use - measurement value before use)/ measurement value before use × 100] Change rateb (%): [(measurement value 21 days after discontinuation - measurement value before use)/measurement value before use × 100] Change ratec (%): [(measurement value 21 days after discontinuation - measurement value after 7 days of use)/measurement value after 7 days of use × 100] | | | | | |

### 5-3) Pore count, ea

- As shown in Table 11 below, it was confirmed that pore count measurement values significantly decreased compared to before using the product, showing an improvement of 10.109 % after 4 days of use, 14.412 % after 7 days of use, and 5.236 % after 21 days of discontinuation, all at a significant level (p<0.05).

**[Table 11]**

| | | **Before use** | **After 4 days of use** | **After 7 days of use** | **21 days after discontinuation** |
|---|---|---|---|---|---|
| **Pore count measurement value (ea)** | **Average** | 96.450 | 86.700 | 82.550 | 91.400 |
| | **Standard** | 28.772 | 30.158 | 29.593 | 30.123 |
| | **Deviation** | | | | |
| **Change rate^{a} (%)** | **Before use - After 4 days of use** | -10.109 | | | |
| | **Before use - After 7 days of use** | -14.412 | | | |
| **Change rate ^{b} (%)** | **Before use - 21 days after discontinuation** | -5.236 | | | |
| **Change rate^{c} (%)** | **After 7 days of use - 21 days after discontinuation** | 10.721 | | | |
| **Significance probability (*p*-value)** | **Before use - After 4 days of use** | 0.001** | | | |
| | **Before use - After 7 days of use** | <0.001** | | | |
| | **Before use - 21 days after discontinuation** | 0.038** | | | |
| | **After 7 days of use - 21 days after discontinuation** | <0.001* | | | |
| **Number of improved test subjects/%** | **Before use - After 4 days of use** | 16 individuals / 80.000 % | | | |
| | **Before use - After 7 days of use** | 19 individuals / 95.000 % | | | |
| | **Before use - 21 days after discontinuation** | 16 individuals / 80.000 % | | | |

| | | | | | |
|---|---|---|---|---|---|
| *** : *P*<0.05 by Paired samples t-test** ****: *P*<0.05 by repeated measures ANOVA** Change ratea (%): [(measurement value after use - measurement value before use)/ measurement value before use × 100] Change rateb (%): [(measurement value 21 days after discontinuation - measurement value before use)/measurement value before use × 100] Change ratec (%): [(measurement value 21 days after discontinuation - measurement value after 7 days of use)/measurement value after 7 days of use × 100] | | | | | |

### 5-4) Pore Volume, mm³

- As shown in Table 12 below, it was confirmed that pore volume measurement values significantly decreased compared to before using the product, showing an improvement of 16.517 % after 4 days of use, 22.823 % after 7 days of use, and 12.012 % after 21 days of discontinuation, all at a significant level (p<0.05).

**[Table 12]**

| | | **Before use** | **After 4 days of use** | **After 7 days of use** | **21 days after discontinuation** |
|---|---|---|---|---|---|
| **Pore volume measurement value (mm³)** | **Average** | 0.333 | 0.278 | 0.257 | 0.293 |
| | **Standard Deviation** | 0.261 | 0.232 | 0.212 | 0.217 |
| **Change rate^{a} (%)** | **Before use - After 4 days of use** | -16.517 | | | |
| | **Before use - After 7 days of use** | -22.823 | | | |
| **Change rate ^{b} (%)** | **Before use - 21 days after discontinuation** | -12.012 | | | |
| **Change rate^{c} (%)** | **After 7 days of use - 21 days after discontinuation** | 14.008 | | | |
| **Significance probability (*p*-value)** | **Before use - After 4 days of use** | <0.001^{##} | | | |
| | **Before use - After 7 days of use** | <0.001^{##} | | | |
| | **Before use - 21 days after discontinuation** | 0.001^{##} | | | |
| | **After 7 days of use - 21 days after discontinuation** | <0.001^{#} | | | |
| **Number of improved test subjects/%** | **Before use - After 4 days of use** | 19 individuals / 95.000 % | | | |
| | **Before use- After 7 days of use** | 20 individuals / 100.000 % | | | |
| | **Before use - 21 days after discontinuation** | 16 individuals / 80.000 % | | | |

| | | | | | |
|---|---|---|---|---|---|
| **^{#}: *P*<0.05 by Wilcoxon signed rank test** **^{##}: *P*<0.025(=0.05/2) by Firedman test, post hoc Wilcoxon signed rank test with Bonferroni correction** Change ratea (%): [(measurement value after use - measurement value before use)/ measurement value before use × 100] Change rateb (%): [(measurement value 21 days after discontinuation - measurement value before use)/measurement value before use × 100] Change ratec (%): [(measurement value 21 days after discontinuation - measurement value after 7 days of use)/measurement value after 7 days of use × 100] | | | | | |

### 6) Subjective survey results based on feelings of use

- The results of the subjective survey evaluation based on the feelings of use after using the test product are shown in the table below.

**[Table 13]**

| | [Survey evaluation after 4 days of use] | | |
|---|---|---|---|
| **Q1** | **Was the attachment method difficult?** | **Number of respondents (individuals) N=20** | **Response rate (%)** |
| **1** | Good | 12 | 60.000% |
| **2** | It was difficult at first, but it becomes easier with practice. | 5 | 25.000% |
| **3** | It seems that a tool to assist with the attachment is needed. | 3 | 15.000% |

| **Q2** | **What is the level of contraction felt upon initial attachment?** | **Number of respondents (individuals) N=20** | **Response rate (%)** |
|---|---|---|---|
| **1** | Level of attachment of Scotch tape | 12 | 60.000% |
| **2** | Level of attachment of nosepack | 7 | 35.000% |
| **3** | Level of hand pinching | 0 | 0.000% |
| **4** | Level of squeezing with whole palm | 1 | 5.000% |
| **5** | No feeling of tightness | 0 | 0.000% |

| **Q3** | **What does the feelings of contraction remind you of? (duplicates are possible)** | **Number of respondents (individuals) N=29** | **Response rate (%)** |
|---|---|---|---|
| **1** | It feels like lifting | 19 | 65.517% |
| **2** | It seems to take the swelling out | 2 | 6.897% |
| **3** | It feels like elasticity is being restored beneath the skin | 6 | 20.690% |
| **4** | It feels like the skin is recovering | 2 | 6.897% |

| **Q4** | **How do you want the contraction to feel?** | **Number of respondents (individuals) N=20** | **Response rate (%)** |
|---|---|---|---|
| **1** | I would like it to be stronger | 11 | 55.000% |
| **2** | Maintain the current level | 9 | 45.000% |
| **3** | I would like it to be weaker | 0 | 0.000% |

| **Q5** | **What is the first change you feel after taking it off?** | **Number of respondents (individuals) N=20** | **Response rate (%)** |
|---|---|---|---|
| **1** | Fine lines have been lightened | 5 | 25.000% |
| **2** | The skin feels more elastic | 6 | 30.000% |
| **3** | The skin feels moisturized | 3 | 15.000% |
| **4** | The skin texture has improved | 2 | 10.000% |
| **5** | There is no change | 4 | 20.000% |

| **Q6** | **How long did such changes last (if you answered questions 1 to 4 in item 2)?** | **Number of respondents (individuals) N=20** | **Response rate (%)** |
|---|---|---|---|
| **1** | Within 10 minutes | 5 | 25.000% |
| **2** | Within 30 minutes | 10 | 50.000% |
| **3** | Within 1 hour | 4 | 20.000% |
| **4** | 4 hours or more | 0 | 0.000% |
| **5** | Half a day or more | 1 | 5.000% |

**[Table 14]**

| | [Survey evaluation after 7 days of use] | | |
|---|---|---|---|
| **Q1** | **What changes are felt in the area where it was attached continuously for 7 days compared to the 4th day (nasolabial folds)?** | **Number of respondents (individuals) N=20** | **Response rate (%)** |
| **1** | Nasolabial folds have lightened | 10 | 50.000% |
| **2** | The skin feels more elastic. | 4 | 20.000% |
| **3** | The skin feels moisturized | 2 | 10.000% |
| **4** | The skin texture has improved. | 0 | 0.000% |
| **5** | Attachment site pores seem to be shrinking | 1 | 5.000% |
| **6** | There is no change | 2 | 10.000% |
| **7** | Other (open-ended response): | 1 | 5.000% |

| **Q2** | **How long did such changes last (if you answered questions 1 to 4 in item 1)?** | **Number of respondents (individuals) N=16** | **Response rate (%)** |
|---|---|---|---|
| **1** | Within 10 minutes | 1 | 6.250% |
| **2** | Within 30 minutes | 3 | 18.750% |
| **3** | Within 1 hour | 7 | 43.750% |
| **4** | 4 hours or more | 4 | 25.000% |
| **5** | Half a day or more | 1 | 6.250% |

| **Q3** | **What changes are felt in the area where it was attached continuously for 7 days compared to the 4th day (forehead)?** | **Number of respondents (individuals) N=20** | **Response rate (%)** |
|---|---|---|---|
| **1** | Noticeable wrinkles have lightened | 6 | 30.000% |
| **2** | The skin feels more elastic | 6 | 30.000% |
| **3** | The skin feels moisturized | 1 | 5.000% |
| **4** | The skin texture has improved | 0 | 0.000% |
| **5** | Eyebrow position is raised | 2 | 10.000% |
| **6** | There is no change | 4 | 20.000% |
| **7** | Other (open-ended response): | 1 | 5.000% |

| **Q4** | **How long did such changes last (if you answered questions 1 to 4 in item 3)?** | **Number of respondents (individuals) N=13** | **Response rate (%)** |
|---|---|---|---|
| **1** | Within 10 minutes | 1 | 7.692% |
| **2** | Within 30 minutes | 4 | 30.769% |
| **3** | Within 1 hour | 3 | 23.077% |
| **4** | 4 hours or more | 3 | 23.077% |
| **5** | Half a day or more | 2 | 15.385% |

**[Table 15]**

| | [Survey evaluation 21 days after discontinuation] | | |
|---|---|---|---|
| **Q1** | **After 7 days of attachment, what has been maintained? (nasolabial folds)** | **Number of respondents (individuals)N=20** | **Response rate (%)** |
| **1** | Nasolabial folds | 6 | 30.000% |
| **2** | Skin elasticity | 7 | 35.000% |
| **3** | Skin texture | 4 | 20.000% |
| **4** | Shrinking pores at the attachment site | 0 | 0.000% |
| **5** | There is no change | 3 | 15.000% |

| **Q2** | **How long did such changes last (if you answered questions 1 to 4 in item 1)?** | **Number of respondents (individuals) N=17** | **Response rate (%)** |
|---|---|---|---|
| **1** | Half a day | 0 | 0.000% |
| **2** | One day | 4 | 23.529% |
| **3** | 2 to 3 days | 4 | 23.529% |
| **4** | One week | 1 | 5.882% |
| **5** | One week or more | 7 | 41.176% |
| **6** | Disappears the day after attachment ends | 1 | 5.882% |

| **Q3** | **After 7 days of attachment, what has been maintained? (forehead)** | **Number of respondents (individuals) N=20** | **Response rate (%)** |
|---|---|---|---|
| **1** | Noticeable wrinkles have lightened | 6 | 30.000% |
| **2** | Skin elasticity | 5 | 25.000% |
| **3** | Skin texture | 3 | 15.000% |
| **4** | Eyebrows raised (eye size increased) | 1 | 5.000% |
| **5** | There is no change | 5 | 25.000% |

| **Q4** | **How long did such changes last (if you answered questions 1 to 4 in item 3)?** | **Number of respondents (individuals) N=15** | **Response rate (%)** |
|---|---|---|---|
| **1** | Half a day | 0 | 0.000% |
| **2** | One day | 4 | 26.667% |
| **3** | 2 to 3 days | 4 | 26.667% |
| **4** | One week | 3 | 20.000% |
| **5** | One week or more | 4 | 26.667% |
| **6** | Disappears the day after attachment ends | 0 | 0.000% |

| **Q5** | **If you were to attach it somewhere else, where would you attach it?** | **Number of respondents (individuals)N=20** | **Response rate (%)** |
|---|---|---|---|
| **1** | Jawline | 5 | 25.000% |
| **2** | Overall area of the cheeks | 7 | 35.000% |
| **3** | Overall forehead/cheeks | 7 | 35.000% |
| **4** | Back of Hand (Hand Care) | 0 | 0.000% |
| **5** | Other (open-ended response) | 1 | 5.000% |

### 7) Safety

No reports of any unusual adverse reactions were received while the test subjects were using the test product, so it is considered a safe product.

In conclusion, the patch according to the present embodiment is judged to help improve elasticity (forehead, cheeks), lifting, wrinkles (forehead, glabella, nasolabial folds), skin density (forehead, cheeks), and pores (area, number, volume) after 4 days of use. After 7 days of use, it is judged to help improve elasticity (forehead, cheeks), lifting, wrinkles (nasolabial folds), density (forehead, cheeks), and pores (depth, area, number, volume).

Additionally, compared to before use, it is judged that 7 days of use and 21 days after discontinuation help improve elasticity (forehead, cheeks), lifting, and pores (area, number, volume). It is also judged that 7 days of use help maintain the improvement in elasticity (cheeks) even 21 days after discontinuation.

### Embodiments

Embodiment 1. A memory film, comprising:
a first layer comprising acrylic acid and polyurethane, the memory film being attached to the skin and then contracting to a memorized rate after a period of time.

Embodiment 2. The memory film of embodiment 1, wherein the memory film enhances skin elasticity,
improves skin wrinkles,
increases skin density, or
reduces one or more of pore depth, pore area, pore count, and pore volume.

Embodiment 3. The memory film of embodiments 1 or 2, wherein the memory film satisfies one or more selected from conditions below:
- the time is 1 to 24 hours
- the memorized ratio is such that a surface area after a period of time is 100 % relative to 150 to 200 % of the surface area at the time of skin attachment.

Embodiment 4. The memory film of any one of embodiments 1-3, wherein the memory film satisfies one or more selected from conditions below:
- the acrylic acid is polyacrylic acid
- a content of the polyurethane may be less than 50 wt% based on a total weight of the first layer.
- a weight ratio of the acrylic acid and polyurethane is 1 to 3: 1
- a total molecular weight of the acrylic acid and polyurethane may be 100,000 to 1,000,000.
- the first layer further comprises Laponite
- the first layer has a thickness of 100 to 3000 µm.

Embodiment 5. The memory film of any one of embodiments 1-4, further comprising:
a second layer comprising one or more selected from the group consisting of polyurethane, spandex, PVC, and nylon.

Embodiment 6. The memory film of any one of embodiments 1-5, wherein the second layer has a thickness of 1 to 200 µm.

Embodiment 7. A use of the memory film of any one of embodiments 1-6, for enhancing skin elasticity; improving skin wrinkles; increasing skin density; or reducing one or more of pore depth, pore area, pore number, and pore volume by attaching the memory film to the skin for 1 to 24 hours.

Embodiment 8. A method of manufacturing the memory film of any one of embodiments 1-6, the method comprising:
applying and drying a solution comprising acrylic acid and polyurethane; and
stretching the applied and dried material.

Embodiment 9. The method of embodiment 8, wherein the solution comprising acrylic acid and polyurethane further comprises one or more selected from the group consisting of Laponite, water, and ethanol.

Embodiment 10. The method of embodiments 8 or 9, further comprising:
drying after the stretching.

## Claims

1. A memory film, comprising:
a first layer comprising acrylic acid and polyurethane, the memory film being attached to the skin and then contracting to a memorized rate after a period of time.

2. The memory film of claim 1, wherein the memory film enhances skin elasticity, improves skin wrinkles,
increases skin density, or
reduces one or more of pore depth, pore area, pore count, and pore volume.

3. The memory film of claims 1 or 2, wherein the memory film satisfies one or more selected from conditions below:
- the time is 1 to 24 hours
- the memorized ratio is such that a surface area after a period of time is 100 % relative to 150 to 200 % of the surface area at the time of skin attachment.

4. The memory film of any one of claims 1-3, wherein the memory film satisfies one or more selected from conditions below:
- the acrylic acid is polyacrylic acid
- a content of the polyurethane may be less than 50 wt% based on a total weight of the first layer.
- a weight ratio of the acrylic acid and polyurethane is 1 to 3: 1
- a total molecular weight of the acrylic acid and polyurethane may be 100,000 to 1,000,000.
- the first layer further comprises Laponite
- the first layer has a thickness of 100 to 3000 µm.

5. The memory film of any one of claims 1-4, further comprising:
a second layer comprising one or more selected from the group consisting of polyurethane, spandex, PVC, and nylon.

6. The memory film of claim 5, wherein the second layer has a thickness of 1 to 200 µm.

7. A use of the memory film of any one of claims 1-6, for enhancing skin elasticity; improving skin wrinkles; increasing skin density; or reducing one or more of pore depth, pore area, pore number, and pore volume by attaching the memory film to the skin for 1 to 24 hours.

8. A method of manufacturing the memory film of any one of claims 1-6, the method comprising:
applying and drying a solution comprising acrylic acid and polyurethane; and
stretching the applied and dried material.

9. The method of claim 8, wherein the solution comprising acrylic acid and polyurethane further comprises one or more selected from the group consisting of Laponite, water, and ethanol.

10. The method of claims 8 or 9, further comprising:
drying after the stretching.
